# EUROPEAN PATENT APPLICATION

(11) **EP 1 018 365 A2**
(43) Date of publication of application: **12.07.2000**
(21) Application number: 00105243.0
(22) Date of filing: 25.01.1996
(51) Int. Cl.: B01J 19/00, G06K 19/06, G09F 3/00

(54) **Coded particles for process sequence tracking in combinatorial compound library preparation**

(30) Priority: 26.10.1995 GB 9521943
(62) Divisional of application: 96935090.9
(71) Applicant: UNIVERSITY OF HERTFORDSHIRE, Hatfield, Herts. AL10 9AB (GB)
(72) Inventor: Kaye, Paul Henry, Hertfordshire SG 4 4QU (GB); Tracey, Mark Christopher, Hertford Heath, Hertfordshire SG 13 7QX (GB)
(74) Representative: Whitaker, Iain Mark

(57) **Abstract**

A solid support particle (3) adapted for use in Combinatorial Chemistry Techniques characterised in that the particle (3) is marked with machine readable code (4, 5, 6, 7, 8).

## Description

### Field of the Invention

This invention relates to particles which are individually and optionally uniquely coded with a machine readable code. One particular application is in the field of Combinatorial Compound Library (CCL) synthesis wherein many compounds are synthesised on small supporting particles or beads, and rapidly screened for desired biological, pharmacological or chemical activity. This application is given by way of illustration only and is in no way limiting. By using the code to identify and record precisely which particles have undergone which chemical reactions, the particular process sequence by which the final compound on each particle was produced may be ascertained.

### Background to the Invention

In the continual quest to reduce research and development costs and to minimise the investment of resources into compounds which may ultimately be unsuitable for the desired end-use, many industries such as the pharmaceutical, agrochemical and biotechnological industries are revolutionising many of their chemical compound synthesis processes. A specific area which has had a major impact over the past five years is that of combinatorial compound libraries (CCL's), a process whereby ensembles of molecular compounds are generated simultaneously (or in a rapid sequence of processes) by combining structural elements from a set of building block molecules onto a common template or scaffold, with highly parallel processing on the entirety of its components [Gordon et al, J. Med. Chem., 1994, 37:1233-1251 and 1386-1401]. The process allows CCL's containing vast numbers of "ligands" (library compounds or elements) to be created in relatively few discrete process steps, albeit with each ligand present in minute quantity. By exposing the library to a biological or chemical system of interest, compounds with specific, desired chemical or pharmacological activity may be expediently identified. Such activity may include for example inhibition or stimulation of an enzyme or pharmacological receptor, the catalysis of a chemical process and the like. This technology is particularly useful when used in conjunction with high throughput screens.

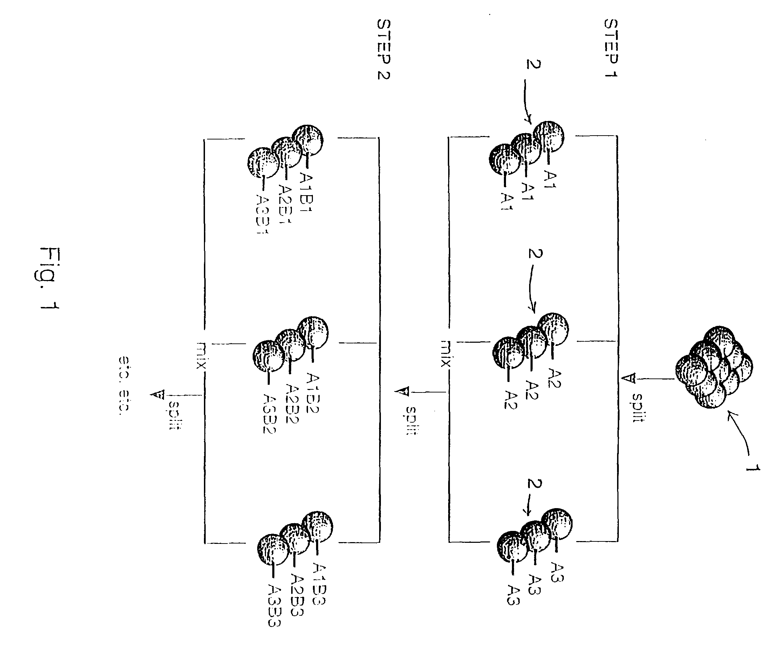

A principal method by which CCL's may be synthesised is based upon the use of minute beads or particles upon which the library of compounds may be produced. The underlying procedure, using sequential mixing, separation, and remixing of discrete compound-carrying particles, was proposed by Furka et al [Abstr. 14th. Int. Congr. Biochem. Prague, 1988, 5:47]. The procedure typically utilises a significant number of small'beads of diameter from a few tens of microns or less to a few hundreds of microns or more which act as the supporting matrices upon which compound synthesis may subsequently take place. The beads are typically of crosslinked polystyrene, polyamide, sintered glass, or similar insoluble material, and possess chemical bonding sites spread throughout the volume of the beads. The procedure for handling the beads is shown schematically in Figure 1.

Typically a suspension of beads, 1, is separated into three separate parts, 2, each of which is then exposed to a specific reagent, Al, A2, or A3 which becomes attached to the beads The three suspensions of beads are then recombined within a single vessel and thoroughly mixed before being divided once again into three separate parts, each part containing a proportion of all three compound types. The large number of beads involved in the process ensures that statistically the numbers of each type of compound in each vessel are approximately equal. The three suspensions are then further exposed to the same or different reagents B1, B2, or B3, and this results, for example in compounds A1B1, A2B1, and A3B1 in the first vessel; A1B2, A2B2, and A3B2 in the second vessel; and A1B3. A2B3 and A3B3 in the third. The suspensions are then remixed in a single vessel and the process repeated. In this way, a substantial number of different compounds may be produced in a relatively small number of reaction steps. At the end of the processing the identity of the compound(s) showing the desired properties may subsequently be deduced.

A difficulty with this "one compound per bead" procedure, especially for non-sequenceable ligands, is that for each discrete bead, its precise history, or more specifically the exact sequence of reagents to which it was exposed, is unknown. Knowledge of this synthetic history is very useful in identifying, discovering or so-called "deconvoluting" the active compound or compounds from a combinatorial chemistry process. One method of generating this information is to follow up each synthesis step with an independent process wherein a chemical "tag" is added to all the beads in that particular vessel [Janda, Proc, Natl. Acad. Sci USA, 1994, 91:10779-85]. through a series of sequential chemical steps, the tags are built up in parallel with the ligands so that at the end of the process the sequence of operations any particular bead has gone through may be retraced by separately analysing the tag sequence. The first tags to be employed in this way were oligonucleotides and peptides [Brenner and Lerner, Proc. Natl. Acad. Sci. USA, 1992, 89:5181-5183; Nikolaiev et al, Peptide Res., 1993, 6:161-170; Needels et al, Proc. Natl. Acad. Sci. USA, 1993. 90:10700-10704]. A further example of this approach is disclosed in PCT WO 93/06121. A disadvantage of this procedure is that these are generally sensitive molecules which are unlikely to be able to withstand some of the harsh reaction conditions (e.g. strong acid, strong base etc ) necessary for the preparation of a broad variety of ligands. Another disadvantage of this procedure is that they have rather limited coding capacity.

More recently a new class of comparatively inert tag molecules has been developed in conjunction with a binary coding system wherein defined mixtures of tag components comprising a combination of haloaromatic and electrophoric moieties are used to represent particular building blocks along with their position within a sequence [Ohlemeyer et al, Proc. Natl. Acad. Sci. USA, 1993, 90:10922-10926]. However, in addition to the problems that arise from the stability associated with chemical tags other important advantages are: the overhead incurred in applying the tag at each process step; the overhead of chemically and/or analytically having to analyse the tag from each, of potentially many, beads which displays the desired activity at the end of the process sequence; and the possibility of artefacts being produced within the tags or ligands by interaction between them.

The present invention, by not relying on the use of chemical tags, overcomes, or mitigates, many of the above limitations. A system has been devised for providing a desired number of particles or solid supports, each with a permanent, machine-readable code, which may be read "on-the-fly" between process steps, thus allowing the process sequence, or audit trail, for each bead to be recorded. Alternatively, if the beads are separated into a multiwell container, for example a 96 well plate such as those routinely used in robotic systems for screening compounds in biological assays, the code may be read when the beads are stationary.

### Summary of the Invention

According to the present invention, there is provided a Combinatorial Chemistry solid support particle marked with a machine readable code, said code being adapted to remain substantially unchanged during Combinatorial Chemistry Library Synthesis, characterised in that the machine readable code is in the form of a binary code and in that at least one dimension of the particle exceeds 50 µm, rendering such a particle visible to the naked eye. For the first time it is possible to track and record a particle of support material as it proceeds through a complex synthetic route involving numerous options.

Preferably the support particle comprises a first phase comprising a solid support suitable for use in Combinatorial Chemistry techniques and a second phase containing a machine readable code. This enables coded microparticle technology to be used in this context.

Preferably, the particle has a bi-layer structure, the first and second phases being in the form of layers superimposed one on another. The two layers are therefore bonded together.

Preferably the second phase incorporating the machine readable code is substantially encapsulated within the first phase, substantially the whole outer surface of the particle thus being free for use as a chemical support. This increases the available area of the support.

In a further embodiment the two phases are mechanically linked.

Preferably the second phase takes the form of a wafer incorporating an aperture and the first phase extends through said aperture such that a portion of the first phase exists on each side of the aperture, the aperture and therefore the wafer, forming a so-called waste in the first phase thus tending to cause the two phases to remain in mechanical contact.

Preferably the second phase incorporates one or more barbed or hook like protrusions adapted to engage the surface of the first phase. This can be likened to a miniature version of hook and loop fastening.

Preferably the support on which chemical synthesis takes place comprises one or more of the following materials:-
porous silicates, for example controlled pore glass; polymeric resin materials for example polystyrene, poly(substituted-styrene), for example poly(halomethylstyrene), poly(halostyrene), poly(acetoxystyrene): polyacrylamides, for example poly(acryloylsarcosine methyl ester); other polyesters, polyacrylates and polymethacrylates; as well as derivatised versions of these resins, for example polystyrene which has been chloromethylated, or poly(acryloylsarcosine methyl ester) wherein the ester has been saponified and the resultant acid derivatised with another moiety of utility in CCL synthesis, as well as optionally cross-linked versions of these resins.

Preferably the code consists of one or more of the following features: pits, holes, hollows, grooves or notches or any combination thereof.

In an alternative embodiment the code resides in the shape of the particle or the shape of the second phase where a second phase is present.

Preferably the machine readable code is readable optically. This enables technology and equipment from related fields to be used.

Preferably the particle further incorporates an orientation marker. This results in increased reliability of results by ensuring that the code is always interpreted correctly.

According to a second aspect there is provided a set of support particles consisting of particles as described above substantially each particle in the set having a unique machine readable code.

In a still further aspect of the invention there is provided a Combinatorial library prepared using the above support particles, regardless of the chemical reactions or sequences used to prepare said library.

The invention also encompasses a method of building and deconvoluting a Combinatorial library comprising the steps of:-
(i) providing a plurality or set of support particles of the type in question;
(ii) suspending said particles in a fluid;
(iii) dividing the fluid containing the particles into a plurality of portions, reading and recording the machine readable codes during or after the division process in order to track the movement of specific particles into respective portions;
(iv) subjecting respective portions to specific chemical reactions;
(v) recombining the respective portions;
(vi) repeating steps (iii), (iv) and (v) as necessary;
so as to create a compound library in which substantially each member of the library is associated with one or more support particles with a machine readable code and tracking data is available to identify the sequence of reactions experienced by substantially each support particle.

There is therefore provided a multiplicity of chemically-inert, micrometer-sized, solid support particles each labelled with a unique, permanent binary code which is machine-readable remotely during the handling of a fluid containing the solid support particles, and each arranged to support and carry a sequence of compounds introduced successively through the fluid in use whereby to build up a labelled compound library Preferably, the code is readable optically, but it is envisaged that alternative forms could be used, provided they did not interfere chemically with the library compounds.

The invention encompasses a compound library which in accordance with the invention is supported on the support particles as defined immediately above. It also provides a method of building a compound library using support particles as defined above comprising the steps of dividing a fluid containing the support particles into portions, whilst reading the labels and tracking the movments of the support particles into the respective portions, allowing the support particles of each portion to combine with and subsequently carry a different respective compound, and repeating the fluid division, tracking and combination steps at least once so as to create a compound library of which each member contains a machine-readable code which can be used with the associated tracking information to identify the sequence of compound combinations and hence the specific compounds.

The present invention thus provides a method for the characterisation and deconvolution of a compound library, which method comprises (i) synthesising the library on a plurality of support particles substantially each particle having an individual, machine-readable code, (ii) testing library compounds in a biological assay, (iii) selecting library compounds of interest, and (iv) identifying such compounds by reference to the code on the associated support particle.

### Brief Description of the Drawings

The invention will be further described, by way of example only, with reference to the accompanying drawings in which:-
Figure 1 illustrates diagramatically a typical procedure for handling beads in a Combinatorial Chemical synthesis sequence;
Figure 2 illustrates typical coded particles according to a first aspect of the present invention;
Figure 3 illustrates cross sections of partially and fully encapsulated coded particles;
Figure 4 illustrates a code reading station;
Figures 5, 6 and 7 illustrate various particle fabrication strategies

### Description of the Preferred Embodiments

The solid support which constitutes the first aspect of the invention conveniently comprises any suitable support material which is inert, that is to say it is of a chemical composition which is not affected by the rigours of library synthesis and testing to any relevant extent. Preferred materials for the synthesis of CCL's according to the method of this invention are porous silicates, for example controlled pore glass, polymeric resin materials for example polystyrene; poly(substituted-styrene), for example poly(halomethylstyrene), poly(halostyrene), poly(acetoxystyrene); polyacrylamides, for example poly(acryloylsarcosine methyl ester); other polyesters, polyacrylates and polymethacrylates; as well as derivatised versions of these resins, for example polystyrene which has been chloromethylated, or poly(acryloylsarcosine methyl ester) wherein the ester has been saponified and the resultant acid derivatised with another moiety of utility in CCL synthesis, as well as optionally cross-linked versions of these resins. The processes for preparing these resins and derivatised versions thereof constitute further aspects of the invention. Furthermore, the above list is not limiting and is given by way of illustration only. It provides examples only of the types of support which are known to the Applicant. Particularly preferred solid support materials according to the method of this invention are polymeric resins, but further suitable solid supports will be apparent to the artisan of ordinary skill

What is important in this particular application is that the support is made of or in part comprises some material, natural or synthetic, which is suitable for use in the chemistry used in Combinatorial Chemistry techniques. This includes materials not yet known or discovered.

A further aspect of this invention is that the individual solid supports each carry a machine-readable code as described hereinbelow. The solid supports are also conveniently in the form of particles, preferably microparticles whose longest dimension is between 1 and 500 microns. Of particular use in compound library synthesis, such as combine/mix/divide processes, are groups of as well as individual microparticles.

Whilst it would be possible to produce microparticles of a desired solid support material or polymer using appropriate micromachining processes, and to mark these particles with identifiable codes, the physical deformation which polymeric supports frequently experience during combinatorial chemical processes, coupled with the low optical opacity typical of the polymer materials employed, may hinder reading of the codes problematic.

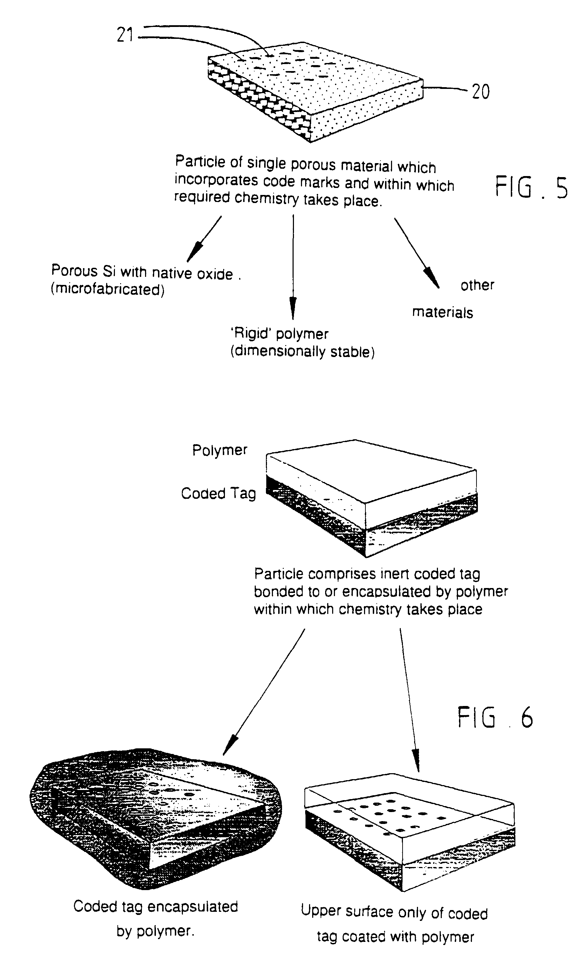

On the other hand, alternative solid support materials, for example specially prepared porous silicon dioxide (glass) particles, which also have utility in the synthesis of combinatorial compound libraries, can, under specific growth conditions, be micromachined to produce particles which can be directly encoded in a manner analogous to that described hereinbelow. However, the process for achieving such low density porous silicon dioxide that would be suitable for CCL synthesis is not presently well understood and though certainly technically feasible, such particles may not be regarded as an optimum embodiment of the invention. This embodiment is illustrated in Figure 5.

Figure 5 illustrates a wafer-shaped particle 20 at least one flat surface of which includes a series of machine readable dots 21. This arrangement has the advantage that only a single solid phase is required and there is no requirement for bonding to dissimilar materials. The particle may incorporate an orientation marker e.g. a corner cut off, shaped groove or the like (not shown) to facilitate accurate reading of the code.

A preferred embodiment of the invention comprises a microparticle which conveniently consists of two distinct phases, one, a solid support material for library synthesis and the other a phase containing a machine-readable code. A particularly preferred embodiment of the invention encompasses a microparticle which comprises one or more of the polymeric solid support materials outlined above, attached to, encapsulating, or otherwise permanently associated with a rigid, opaque, coded subparticle, and which combines the desired properties of both the polymeric solid support material and the coded microparticle. This principle is illustrated in Figure 6. Such individually coded particles are then useful in combinatorial processes to allow the tracking of each particle through the various chemical processing steps, the particle codes being machine-read 'on-the-fly' as the particles move from one process step to another or after completion of the screening process.

By way of non-limiting examples, a description of the subparticles of the second phase which constitute a further aspect of the invention is given below.

Code marks may be applied to rigid materials such as silicon, silicon dioxide, or a metal, using the process of micromachining. These materials can then be further micromachined into particles of defined size and shape. Such materials offer the additional advantage of comparative robustness and, in certain cases, chemical inertness. By way of exemplification, it has been shown by Kaye et. al. in the Journal of Aerosol Science Vol. 23 [1992, Supplement 1, 201-204] that extremely uniform particles of silicon or silicon dioxide (glass) can be made using the technology of micromachining. Particles may also be manufactured from metals such as aluminium or gold, as well as polyamide or other polymeric or resin materials. Preferred materials for the manufacture of subparticles are silicon and silicon dioxide. The machining technology was developed from the microelectronics industry and uses similar processes of deposition and etching to those used to make microelectronic integrated circuits. The manufacture of such particles involves typically:- the design of the required particle geometry (or geometries) using computer aided design (CAD) tools; the manufacture of appropriate photolithographic masks which delineate the particle shapes; the deposit onto a previously prepared silicon wafer substrater i.e. coated with a sacraficial layer and (typically 3, 4 or 8 inches in diameter) of a layer of, for example, silicon or silicon dioxide from which the particles will be made; the coating of this layer with a photosensitive polymer resist which, upon ultraviolet exposure through the photographic mask, defines the particle shapes; and finally the creation of the particles on the wafer substrate by removing unexposed resist and etching away the revealed inter-particle areas of silicon or silicon dioxide. The particles may then be freed from the wafer substrate by dissolution of a sacrificial layer (typically made of aluminium if the particles are silicon or silicon dioxide) which underlies the particles. Subparticles can be designed to have widths from a few microns to greater than 100 microns and thicknesses up to the order of 10 microns.

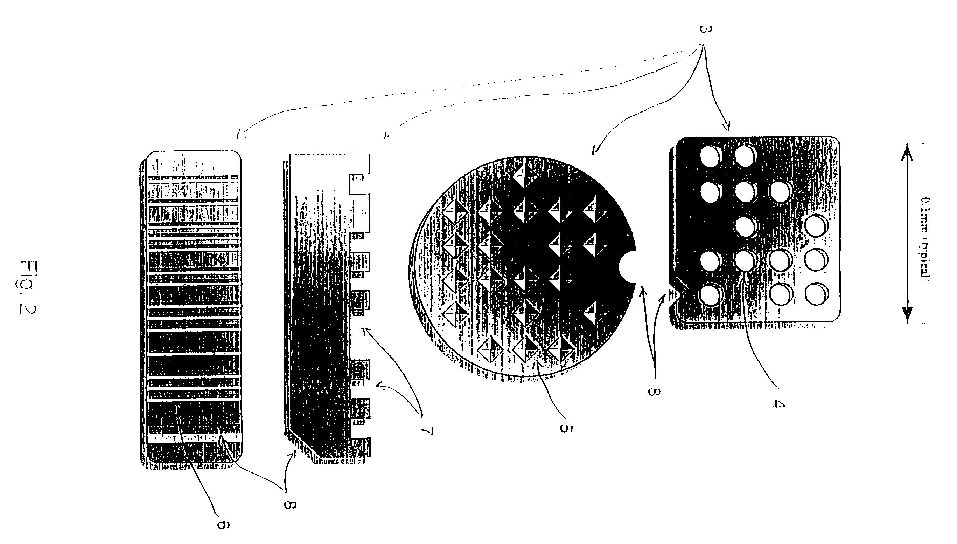

Such is the accuracy and high resolution of the CAD and photolithographic processes which define the particle shapes, that it is possible additionally to design the subparticles, 3, (see Fig. 2) to carry marks such as etched-through holes, 4, etched pits, 5, or etched grooves, 6, which constitute a unique code, typically in binary format. The subparticle outlines can also be notched, 7, to indicate a code, and orientation marks, 8, can be included to prevent code misreading. This process is the subject of a separate patent application GB-A- 2289150; filed on 25 April 1994 naming as inventors Kaye, P.R., Tracey, M.C. and Gordon, J. A. For the avoidance of doubt the entire text of GB 2289150 is hereby imported by reference. This text and the associated diagrams are intended to be an integral part of this present application and the inventive concepts herein described. A wafer substrate may thus be etched by micromachining processes to yield typically a million or more particles, each, if desired, carrying a different binary code. The microscopic code on the particles may be interrogated and read using appropriate microscope-based image processing systems. By way of example, a code containing just twenty binary sites (pits, holes, or similar devices) would allow a million particles to be uniquely numbered from 1 to 1,000,000.

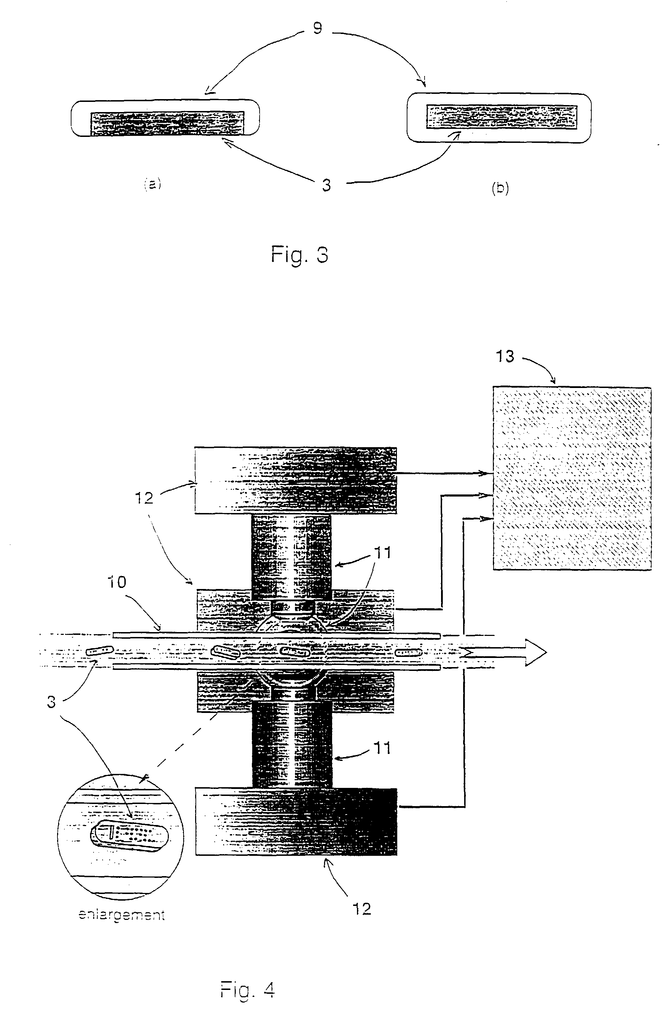

Figures 3a and 3b show preferred embodiments of the polymer-coated, micromachined subparticles - the microparticles of the invention. In Fig. 3a the polymer coating, 9, is applied principally to one surface the upper surface of the micromachined subparticle as illustrated, 3, to give a bilayer microparticle. In Fig. 3b the subparticle, 3, is encapsulated within the polymer coating, 9, to give a cored microparticle.

A preferred, but non-limiting method of producing single-surface coated microparticles is as follows. The silicon or silicon dioxide particles are etched on a silicon wafer substrate of say 4 inches diameter as described by Kaye et. al (vide supra). The particles are etched following exposure through an appropriate photolithographic mask such that each particle carries a unique binary, ternary or other code. Before the particles are released from the wafer substrate (by the process described by Kaye), an additional layer of the desired polymer is applied across the entire wafer. The thickness of the layer would typically be of the order of 10 to 30 microns. The layer would be deposited in a similar way to normal photosensitive polymer photoresists which are routinely used in the microelectronics and micromachining technologies. This involves applying a thick layer of the polymer (either molten polymer or a polymer thinned with appropriate solvent) to the wafer which is then spun at high speed about the axis orthogonal to its surface. The outward centrifugal flow of the polymer results in uniform thinning of the layer to the desired depth. Once hardened, the polymer layer is coated with a further photoresist layer and this is exposed through an additional photolithographic mask to define discrete areas covering each of the coded micromachined particles. The unexposed resist is subsequently removed by a photoresist-specific solvent, exposing the inter-particle areas of polymer layer which may then be removed by a suitable polymer dissolving solvent. The silicon, or silicon dioxide/polymer microparticles are finally released from the wafer by dissolution of the sacrificial aluminium layer underlying them.

One preferred, but non-limiting, method of producing fully polymer-coated microparticles is as follows. The coded micromachined silicon or silicon dioxide particles are freed from the wafer in the manner described by Kaye et. al. These subparticles are then suspended in a melt or solution of the polymer to be applied as a coating. The suspension is then sprayed with sufficient pressure through a fine orifice to produce a droplet jet. Under the correct fluid dynamic conditions, determined by experimentation, the micromachined particles encapsulated in a layer of polymer will be ejected singly from the orifice. Upon solidification or hardening, the polymer coated, coded microparticles may be separated from the remainder by centrifugation in an aqueous suspension (the coded silicon particles being of greater density than the polymer would cause the required coated particles to precipitate below the homogeneous polymer beads), or by suspension in an organic solvent which caused the polymer to swell. The coated particles, though also exhibiting polymer swelling, would be less buoyant than the homogeneous polymer particles (which float to the surface of the suspension) and would sink, facilitating their removal.

A second preferred, but non-limiting method involves preformed silicon or silicon dioxide subparticles being introduced into a heterogeneous aqueous mixture of monomer precursors, for example styrene, halomethylstyrene, halostyrene, acryloylsarcosine methyl ester etc., or mixtures thereof, along with, optionally, crosslinking agents as well as such polymerisation catalysts and initiators as would be known to any person skilled in the art, such that, under controlled conditions of emulsion polymerisation, the polymer forms, encapsulates the subparticles and generates the microparticles. The microparticles are then recovered by filtration and washed copiously to remove all by-products of the polymerisation reaction.

A particularly preferred material for the production of coded subparticles is silicon because of its opacity at visible wavelengths and inertness to most chemicals used in the processes of combinatorial chemistry. Particularly preferred machine readable codes carried by the subparticles are pits, holes, grooves, notches, or combinations thereof. The codes are also preferably binary in nature. It is also particularly preferred that the subparticles carry some form of micromachined orientation marking, for example one or more grooves or holes, in order to render the microparticles non-symmetrical and thus to assist the code reading process.

A further embodiment of this application comprises subparticles which are derived from a non-porous, rigid, inert material such as silicate (glass) into which has been deposited, during or after production, but before association with the polymeric solid support, a suitable number of individual materials which can be altered by application of an external non-chemical stimulus, for example electromagnetic radiation. By virtue of the changes imbued in them by the particular stimulus, these materials can, by suitable readout of the residual deposits, reveal the process sequences that those particles/subparticles have experienced. Non-limiting examples of such materials include fluorescent and phosphorescent chemicals, which can be selectively modified by light of a specific wavelength, and also detected by a machine capable of detecting transmitted, refracted or reflected light at one or many wavelengths.

Alternatively, the material deposited within the subparticle may not be modifiable. The information that such material can reveal would pertain to the very first process that the particular particle/subparticle had experienced. By way of non-limiting example, if one or more heavy metals were to be incorporated into a glass subparticle such that each metal element or combination of metals were to be indicative of a particular building block to be added in the first stage of the combinatorial synthesis (for example, in Figure 1, copper might be the "tag" for building block 'A1'), then any active or interesting ligand, as defined herein, which is associated with a bead that, upon analysis by, for example atomic absorption spectroscopy, reveals copper to be present, can be inferred to have originated from the process that involved the chemical process aimed at introducing building block A1.

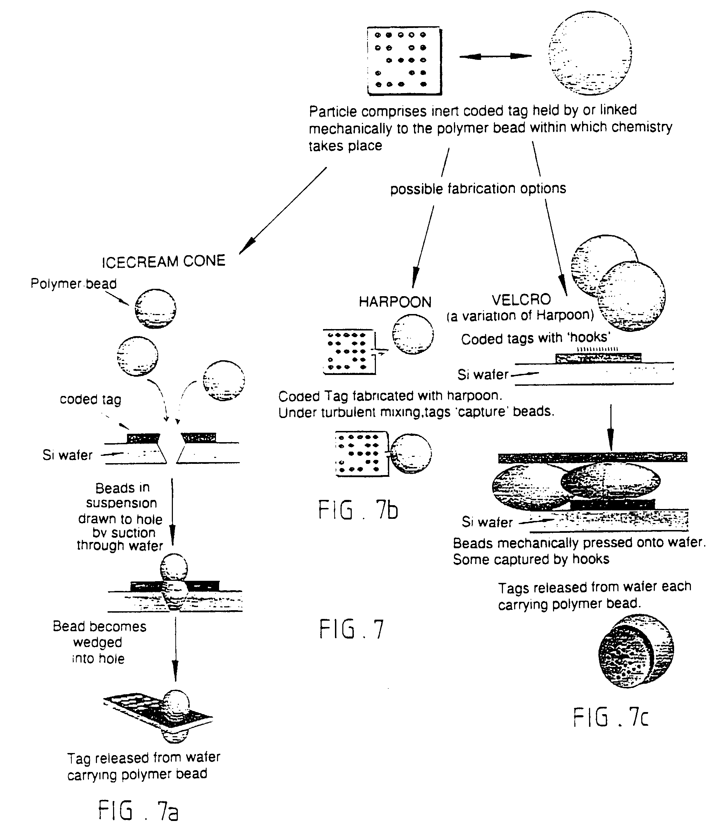

In another embodiment of the invention, the coded microparticle may be linked to the appropriate polymer mechanically by a variety of methods. Numerous methods of fabrication of such a mechanically linked assembly are possible. In one method as illustrated in Fig 7a the polymer beads are suspended in an appropriate liquid, e.g. water or a solvent, and drawn by suction through an appropriately shaped hole in the coded tag as shown. The tag is released from the silicon wafer as described previously.

In another method Fig. 7b, the coded tag is fabricated in such a way that a hook or harpoon is incorporated. Under turbulent mixing the polymer bead will become attached. In these methods it may be advantageous to soften the polymer by exposure to solvent before carrying out these operations.

In a further method as illustrated in Fig 7c where the coded tag is still attached to a silicon wafer, the polymer beads may be attached to the coded tag bearing one or more hooks by mechanically pressing the beads against the tags with an appropriate flat surface. The tag is released from the silicon wafer as described previously.

In the methods 7a, 7b and 7c described above a 1:1 ratio of coded tag and polymer bead results. In order to increase the surface area of polymer available for synthesis in CCL it would be possible to increase the number of polymer beads attached to a coded tag by increasing the number of holes or hooks in the devices described above.

Figures 7b and 7c illustrate just two possible methods by which a support particle suitable for carrying out Combinatorial Chemistry can be mechanically linked to a coded particle. These methods are intended as illustrations only. This invention is intended to encompass all methods of mechanical fixing, be they temporary or permanent.

For the first time it has been possible to give support particles of the type in question a unique code in order to follow, monitor, track and record the progress of an individual particle through a complex sequence of operations.

The strength and mechanical stability of the link between the two phases may be adjusted according to the desired conditions of use.

The geometry of the coded phase will depend on the shape of the first phase, the nature of the coded information amongst other factors.

In a particularly preferred embodiment of this application, each coded microparticle possesses a unique code. By this means a CCL of, for example, a million compounds can be represented by a million unique coded microparticles using a binary code of 20 binary sites, or a CCL of, for example, 100,000 elements can be represented by a million coded microparticles using a code of 20 binary sites such that each compound is represented, redundantly, ten times. The key advantages of having many individually labelled, discrete solid support particles at the outset of the CCL synthesis are, amongst others:- the avoidance of having to perform extra chemical steps at each stage of the chemical process - each code or tag is present from the outset; the avoidance of having to analyse chemically the tag from each bead of interest - a simple reading of the solid state code contained within the microparticle by a process outlined hereinbelow immediately reveals the history of that particular bead; and the avoidance of any artefacts being produced within the tags or ligands by interaction between them - the code is inert and is carried by an inert material.

The process as exemplified above of assigning a unique code to each and every process path within a combinatorial chemical library synthesis, and thus by inference, each and every putative compound that is contained within that defined CCL, combined with the ability easily to read that code displayed by any solid support particle both during the chemical process sequence and after a defined selection process, is a particularly important and novel aspect of this invention.

Furthermore, the combinatorial compound library may comprise any convenient number of individual members, for example tens to hundreds to thousands to millions etc., of suitable compounds, for example peptides, peptoids and other oligomeric compounds (cyclic or linear), and template-based molecules, for example benzodiazepines, hydantoins, biaryls, polycyclic compounds (e. g. naphthalenes, phenothiazines, acridines, steroids etc.), carbohydrate and amino acid derivatives, dihydropyridines, benzhydryls and heterocycles (e.g.. triazines, indoles etc.). The numbers quoted and the types of compounds listed are illustrative but not limiting.

The compound library preferably comprises chemical compounds of low molecular weight and potential therapeutic agents. Such compounds are for example of less than about 1000 daltons, such as less than 800, 600 or 400 daltons.

Combinatorial Libraries are intended for testing in a variety of assays. The purpose of any assay is to test the ability of library elements or compounds to modulate activity in a test system of interest. Particularly preferred, but in no way limiting, are biological assays, biological systems and biologicals of interest including high throughput screens. Convenient biologicals of interest include proteins such as enzymes, receptors, signalling systems, reporter genes and the like. Suitable test systems will be apparent to the scientist of ordinary skill. Any convenient number of library compounds may be tested in the biological assay.

Synthesis of the compound library on the microparticles may comprise any convenient number of individual reaction steps. Chemical libraries synthesised on a plurality of coded microparticles as hereinbefore defined are novel and represent further and independent aspects of this invention.

Furthermore, the coded microparticles as herein described form further and independent aspects of the invention. In order satisfactorily to track particles through the multi-stage processing of combinatorial chemistry, it is necessary to be able to record the code from each particle as it passes from one reaction vessel to another between process steps. Whilst this could be achieved by collecting the particles and observing them whilst stationary (using a microscope based image capture and processing system), the preferred embodiment is for the particle codes to be read on-the-fly. Alternatively, if the beads are separated into a multiwell container, for example a 96 well plate, such as used in robotic systems used for screening compounds in biological assays, the code may be read when the beads are stationary.

Figure 4 shows an embodiment of a code reading station. It comprises a capillary flow channel, 10, carrying in suspension the coded microparticles, 3, between process steps, and a reading station, 11, which incorporates a microscope objective lens system and which is capable of acquiring an image or images of each particle as it traverses the reading station. Each image may then be processed electronically by a computing system, 13, to identify the code. The desired particle flow may be achieved by applying over-pressure to the upstream reaction vessel, by manual pipetting, or by some other technique. At one point the flow passes through a transparent channel, 10, whose dimensions are such that the particles tend to align in single file as they pass through the channel. This type of particle trajectory control may be achieved simply by appropriate channel design, although the use of laminar flow focusing (commonly used in flow-cytometers to align biological cells in liquid flow) would be preferred. Furthermore, by designing the particles to have a reasonably high aspect ratio of length to width (typically 5:1), the particles can be made to travel along the channel with their long axis preferentially aligned with the axis of flow, thus facilitating observation of the codes. Hence, a particularly preferred embodiment of the invention encompasses coded microparticles with a suitable aspect ratio of length to width of between 3:1 and 10:1. By way of non-limiting example, a "lozenge"-shaped microparticle of 150 microns length would preferably possess a width dimension of between 15 and 50 microns, and one of 300 microns length, a width of between 30 and 100 microns.

The axial rotation of each individual particle is more difficult to control and therefore the code reading station, 11, must be capable of observing the particle from several directions simultaneously. This could be achieved either by the use of mirrors at either side of the channel reflecting images to a single objective lens system, or preferably by the use of four orthogonally arranged objective lens imaging systems (see Fig.4). The image(s) of the particle may be captured via a microscope objective lens system by conventional miniature charge-coupled-device (CCD) cameras 12. Particularly preferred is monochromatic illumination from, for example, a diode laser, which may improve the contrast ratio in the acquired image.

The captured image(s) from each particle may then be processed by a dedicated computing system, 13, using either conventional commercial image processing software such as Optimas (TM) or Visilog (TM), or preferably dedicated custom software which could be programmed to provide desirable speed advantages. The processing would deduce the orientation of the particle and ascertain the positions of the code marks on the particle. From this, the complete code would be determined and recorded. With currently available computer processing power, particle codes would be read at maximum rates of typically 50 to 100 per second. This rate will be dependent on the particular application and technology available. The use of two reading stations in series along a channel would allow checking of code data and minimise the possibilities of misread codes due to particle coincidences within the measurement zone.

For the encapsulated particle types, it is important that the polymer coating is sufficiently transparent for the code on the particle to be successfully read. Most polymers are translucent rather than transparent at visible wavelengths, and therefore the thickness of the coating should not be such that the code information is unreadable. Preferred polymer thicknesses are between 5 and 50 microns. Particularly preferred thicknesses are between 10 and 30 microns. By way of non-limiting example, an optically acceptable thickness of 15 microns would, when coating a silicon particle of, for example, 120 microns x 25 microns x 10 microns in size, constitute a total polymer volume equivalent to that of a 130 microns diameter solid polymer bead, and would be quite suitable for use in combinatorial chemistry. The code information recorded at each of many reading stations arranged between the chemical process vessels would be collated on a central computer system and be available for interrogation to ascertain the specific route each coded microparticle had taken through the reaction processes.

It will be appreciated that the methods, materials and techniques described above can be applied in other situations where it is required to monitor an individual item through a complex series of operations.

## Claims

1. A combinatorial Chemistry solid support particle marked with a machine readable code, said code being adapted to remain substantially unchanged during Combinatorial Chemistry Library Synthesis.

2. A support particle as claimed in Claim 1 wherein the support particle comprises a first phase comprising a solid support suitable for use in Combinatonal Chemistry techniques and a second phase containing the machine readable code.

3. A support particle as claimed in Claim 2 wnerein the particle has a bi-layer structure the first and second phases being in the form of layers superimposed one on another.

4. A support particle as claimed in Claim 2 wherein the second phase incorporating the machine readable code is substantially encapsulated within the first phase substantially the whole outer surface of the particle thus being free for use as a chemical support.

5. A support particle as claimed in Claim 2 wherein the two phases are mechanically linked.

6. A support particle according to Claim 5 wherein the second phase takes the form of a wafer incorporating an aperture and the first phase extends through said aperture such that a portion of the first phase exists on each side of the aperture the aperture and therefore, the wafer, forming a so-called waist in the first phase thus tending to cause the two phases to remain in mechanical contact.

7. A support particle as claimed in Claim 5 wherein the second phase incorporates one or more barbed or hook like protrusions adaptd to engage the surface of the first phase.

8. A support particle as claimed in any preceding claim wherein the support on which chemical synthesis takes place comprises one or more of the following materials:-
porous silicates, for example controlled pore glass; polymeric resin materials for example polystyrene, poly(substituted-styrene), for example poly(halomethylstyrene), poly(halostyrene), poly(acetoxystyrene); polyacrylamides, for example poly(acryloylsarcosine methyl ester); other polyesters, polyacrylates and polymethacrylates; as well as derivatised versions of these resins, for example polystyrene which has been chloromethylated, or poly(acryloylsarcosine methyl ester) wherein the ester has been saponified and the resultant acid derivatised with another moiety of utility in Combinatorial Chemistry Library synthesis, as well as optionally cross-linked versions of these resins.

9. A support particle as claimed in any preceding Claim wherein the code consists of one or more of the following features: pits, holes, hollows, grooves or notches or any combination thereof.

10. A support particle as claimed in any preceding Claim modified in that the machine readable code resides in the shape of the particle or the shape of the second phase where a second phase is present.

11. A support particle as claimed in any preceding Claim wherein the machine readable code is readable optically.

12. A support particle as claimed in any preceding Claim wherein the particle further incorporates an orientation marker.

13. A set of support particles consisting of particles as claimed in any preceding claim, substantially each particle in the set having a unique machine readable code.

14. A Combinatorial library prepared using support particles according to any preceding Claim, regardless of the chemical reactions or sequences used to prepare said library.

15. A method of building and deconvoluting a Combinatorial library comprising the steps of:-
(i) providing a plurality or set of support particles according to any of the claims 1-13 incusive;
(ii) suspending said particles in a fluid;
(iii) dividing the fluid containing the particles into a plurality of portions, reading and recording the machine readable codes during or after the division process in order to track the movement of specific particles into respective portions;
(iv) subjecting respective portions to specific chemical reactions;
(v) recombining the respective portions;
(vi) repeating steps (iii), (iv) and (v) as necessary.;
so as to create a compound library in which each member of the library is associated with one or more support particles with a machine readable code and tracking data is available to identify the sequence of reactions experienced by substantially each support particle.

16. A method for the characterisation and deconvolution of a compound library comprising the steps of:-
(i) synthesising a compound library on a plurality of solid support particles, each particle being as claimed in any of claims 1 to 13 inclusive;
(ii) testing said library of compounds in a biological assay;
(ii) selecting library compounds of interest; and
(iv) identifying such compounds by reference to the code on the associated support particle.
